(19) **Europäisches Patentamt / European Patent Office / Office européen des brevets**

(11) **EP 1 219 708 B1**

(12) **EUROPEAN PATENT SPECIFICATION**

(45) Date of publication and mention
of the grant of the patent:
**02.05.2007 Bulletin 2007/18**

(51) Int Cl.:
*C12N 15/11* (2006.01)     *A61K 31/70* (2006.01)
*C12Q 1/68* (2006.01)      *G01N 33/15* (2006.01)

(21) Application number: **00912930.5**

(22) Date of filing: **29.03.2000**

(86) International application number:
**PCT/JP2000/001969**

(87) International publication number:
**WO 2001/027263 (19.04.2001 Gazette 2001/16)**

(54) **MODULATE APTAMER AND METHOD FOR DETECTING TARGET PROTEIN BY USING THE SAME**

MODUL-APTAMER UND VERFAHREN ZUR DETEKTION VON ZIELPROTEINEN DURCH
ANWENDUNG DESSELBEN

APTAMERE MODULE ET PROCEDE DE DETECTION D'UNE PROTEINE CIBLE A L'AIDE DE CELUI-
CI

(84) Designated Contracting States:
**DE FR GB**

(30) Priority: **08.10.1999 JP 28867799**

(43) Date of publication of application:
**03.07.2002 Bulletin 2002/27**

(73) Proprietor: **National Institute of Advanced
Industrial Science
and Technology
Tokyo 100-8921 (JP)**

(72) Inventors:
• **KUMAR, Penmetcha
Tsukuba-shi, Ibaraki 305-0044 (JP)**
• **YAMAMOTO, Rika
Saitama 351-0115 (JP)**

(74) Representative: **Maschio, Antonio
D Young & Co
120 Holborn
London EC1N 2DY (GB)**

(56) References cited:
WO-A-93/06122          JP-A- 11 127 864
US-A- 5 527 894

• **YAMAMOTO R. ET AL.: "Isolation and
characterization of an RNA that binds with high
affinity to Tat protein of HIV-1 from a completely
random pool of RNA" GENE THERAPY AND
MOLECULAR BIOLOGY, vol. 1, March 1998
(1998-03), pages 451-466, XP008031759**
• **TYAGI S. ET AL.: 'Molecular Beacons: Probes that
Fluoresce upon Hybridization' NATURE
BIOTECHNOLOGY vol. 14, no. 3, 1996, pages 303
- 308, XP002929045**
• **T. YAMAMOTO ET AL.: 'Shinki Kakusan Iyakuhin;
Peptide Iyakuhin no Shinkahou ni yoru Tansaku'
IDENSHI IGAKU vol. 2, no. 3, 1998, pages 375 -
380, XP002946300**

Note: Within nine months from the publication of the mention of the grant of the European patent, any person may give
notice to the European Patent Office of opposition to the European patent granted. Notice of opposition shall be filed in
a written reasoned statement. It shall not be deemed to have been filed until the opposition fee has been paid. (Art.
99(1) European Patent Convention).

**Description**

TECHNICAL FIELD

The present invention relates to a modulate aptamer which binds specifically to a specific target protein, in particular, Tat protein of human immunodeficiency virus type-1 (HIV-1), and a method and a kit for detecting a target protein, in particular HIV-1 Tat protein, using the same.

BACKGROUND ART

It has been reported that among nucleic acid ligands (aptamers), there are those having affinity/specificity for a protein comparable to the affinity/specificity of an antibody for its antigen, and it is expected that these can be used as a molecule recognition factor in a biosensor. (Osborn, S.E. and Ellington, A.D., *Chem. Rev.* 97, 349-370 (1997)). To this end, research conducted using full-length aptamer has been promoted and carried out (Drolet, D.W., Moon-McDermott, L. and Roming, T.S. *Nature Biotechnol.* 14, 1021-1025 (1996); Kleinjung, F. et al, *Anal. Chem.* 70, 328-331 (1998); Potyrailo, R.A., Conrad, C.R., Ellington, A.D. and Hieftje, G.M. *Anal. Chem.* 70, 3419-3425 (1998)).

Further, it was thought that an aptamer exhibiting high affinity with Tat protein of HIV-1 and HIV-2 has sensitivity sufficient to measure the amount of Tat protein released from infected cells or in the sera of infected patients. (Westendrop, M.O. et al, *Nature* 375, 497-500 (1995); Pantaleo, G. et al, *Nature* 362, 355-358 (1993); Embretson, J. et al, *Nature* 362, 359-362 (1993)).

Yamamoto et al., *Gene Therapy and Molecular Biology* 1, 451-466 (1998) describes isolating and characterising RNA molecules that bind with high affinity to Tat protein of HIV. This document also reports that Tat protein binds with high affinity to annealed RNA molecules containing no stem-loop structures.

Recently, a nucleic acid motif having a stem-loop structure called a molecular beacon was developed as a tool for finding complementary target sequences (Tyagi, S. and Kramer, F.R. (1996) Nature Biotechnology 14, 303-308). This molecular beacon includes two structural components: a loop sequence being a probe complementary to a target sequence, and a stem structure formed by annealing of complementary arm sequences on the both ends of the probe sequence. A fluorescent probe is attached to the end of one arm forming the stem structure by a covalent bond, and a quencher substance is attached to the end of the other arm by a covalent bond. The fluorescent probe and quencher substance are in close proximity due to the stem formation of this beacon, and as a result, fluorescence is not emitted. When this molecular beacon encounters a target molecule, it forms a probe (loop structure)-target hybrid that is more stable than the stem structure of the molecular beacon. As a result of a structural change in the stem-loop motif, the two arm sequences move away from one another, enabling fluorescence to be emitted. Therefore, using a molecular beacon, it is possible to detect a specific nucleic acid in real-time without stopping the reaction. Further, it is reported that this can be applied to live cells. (Tyagi, S. and Kramer, F.R. (1998) Nature Biotechnology 16, 49-53; Matsuo, T. (1998) Biochem. Biophys Acta. 1379, 178-184; Sokol, D.L., Zhang, X., Lu, P. and Gewirtz, A.M. (1998) Proc Natl. Acad. Sci. USA 95, 11538-11543).

However, there were a number of limitations to this first approach described above. Examples of such limitations include the fact that the longer the length of the aptamer, the lower the efficiency of chemical synthesis; the necessity of protecting the full-length aptamer against nuclease; and the fact that in the case of a plurality of analyses, with long aptamers the structuring/restructuring efficiency is low. That is, for reasons of the size and recognition mode of aptamers, there was a limit to the application of aptamers to experiments using biosensors.

DISCLOSURE OF THE INVENTION

The present inventors solved the above problem, and developed a novel strategy which enables use as an effective biosensor by using an aptamer which forms a conjugate and stabilizes only in the presence of a target protein or substance to be analyzed. Specifically, based on an aptamer RNA for an HIV-1 Tat protein which was the subject of an earlier patent application (Japanese Patent Laid-Open Application No. Hei 11-127864), the aptamer sequence was divided onto independent double strands and a modulate aptamer more effective in detection of Tat protein was constructed.

It was found that the HIV-1 Tat aptamer RNA-derived modulate aptamer according to the present invention forms a conjugate in the presence of HIV-1 Tat protein or Tat-derived peptide at 1 nM or less, whereas it does not form a conjugate in the presence of other RNA-binding proteins or nuclear extracts.

That is, the present invention relates to a modulate aptamer being an aptamer constituted by two complementary oligonucleotide chains which forms a conjugate and stabilizes only in the presence of a target protein, and particularly relates to the modulate aptamer for which the target protein is HIV-1 Tat protein and/or a fragment thereof.

As an example of the modulate aptamer of the present invention, a modulate aptamer having the nucleotide sequence represented by the following secondary structure (I) is provided.

$$
\begin{array}{c}
3' - N^{6a} - N^{6b} \quad - \quad 5' \\
C \ - \ G \\
N^{5a} - N^{5b} \\
\qquad\qquad U \\
\qquad\qquad\quad N^4 \\
C \ - \ G \\
U \ - \ A \\
A \ - \ U \\
G \ - \ C \\
N^3 \\
U \\
\qquad N^{2a} - N^{2b} \\
G \ - \ C \\
5' - N^{1a} - N^{1b} \ - \ 3'
\end{array}
\qquad (\mathrm{I})
$$

(In the structure, $N^{1a}$ and $N^{1b}$ represent at least 1 pair of nucleobases capable of complementary base pair formation; $N^{2a}$ and $N^{2b}$ represent at least 1 pair of nucleobases capable of complementary base pair formation; $N^3$ and $N^4$ each independently represent 1 or 2 nucleobases; $N^{5a}$ and $N^{5b}$ represent at least 1 pair of nucleobases capable of complementary base pair formation; $N^{6a}$ and $N^{6b}$ represent at least 1 pair of nucleobases capable of complementary base pair formation; and solid lines represent hydrogen bonds between nucleobases.)

As a particularly preferable modulate aptamer according to the present invention, a modulate aptamer having the nucleotide sequence represented by the following secondary structure (II) is provided.

$$
\begin{array}{c}
3' \qquad 5' \\[4pt]
G - C \\
A - U \\
G - C \\
C - G \\
C - G \ U \\
C - G \ C \\
U - A \\
A - U \\
{}_U G - C \\
{}^U C - G \\
G - C \\
A - U \\
A - U \\
G - C \\[4pt]
5' \qquad 3'
\end{array}
\qquad (\mathrm{II})
$$

(In the structure, solid lines represent a hydrogen bond between nucleobases.)

Further, using the above-mentioned modulate aptamer, a method enabling detection of a target protein such as an HIV-1 Tat protein, simply and with high sensitivity, specifically, a micro-titer plate assay using fluorescence, was developed. It is thought that there is the possibility that this assay can be used in a manner similar to a DNA array, and can be applied and extended to detection of virus proteins and other small molecule substances.

Further, as one embodiment of the present invention, there is provided a modulate aptamer in which one of the oligonucleotide chains constituting the modulate aptamer has, intramolecularly, mutually complementary sequences of four or more consecutive nucleotides, and which adopts a stem-loop structure in the absence of a target protein.

Specifically, for example, when a fluorescent substance is bound to the 5' or 3'-end of the oligonucleotide chain of the stem-loop structure, and a quencher substance in respect of the fluorescent substance is attached to the 3'or 5'-end, respectively, such that it forms a conjugate only in the presence of a target protein and emits fluorescence, then detection becomes very easy.

As a preferable oligonucleotide chain of this stem-loop structure, one which has the nucleotide sequence represented by the following secondary structure (III) is provided.

$$(III)$$

(In the structure, solid lines represent hydrogen bonds between nucleobases.)

This novel strategy for "modulate aptamers" can be easily applied to a biosensor for characterizing molecule recognition factors for various molecules.

Therefore, the present invention further provides a method of detecting a target protein, particularly an HIV-1 Tat protein and/or fragment thereof, which comprises radioactively or non-radioactively labeling one oligonucleotide chain of the modulate aptamer, and detecting the presence and/or amount of the target protein with the presence or absence, and/or amount of a conjugate formed in the presence of the target protein.

In one embodiment of the method of the present invention, the non-radioactive label is fluorescein, and the conjugate is detected by a fluorescent signal thereof.

Further, the present invention provides a method of detecting a target protein, particularly, an HIV-1 Tat protein and/or fragment thereof, which comprises immobilizing one oligonucleotide chain of the modulate aptamer on a support, and detecting the presence and/or amount of the target protein with the presence or absence, and/or amount of a conjugate formed by addition of the other oligonucleotide chain labeled radioactively or non-radioactively.

In one embodiment of the method of the present invention, the immobilization is by specific binding between avidin or streptoavidin and biotin.

According to the present invention, there is further provided a kit for detecting a target protein, particularly, an HIV-1 Tat protein and/or fragment thereof, wherein the kit comprises a support, one oligonucleotide chain of the modulate aptamer to be immobilized on the support, and the other oligonucleotide chain which forms a conjugate in the presence of the target protein.

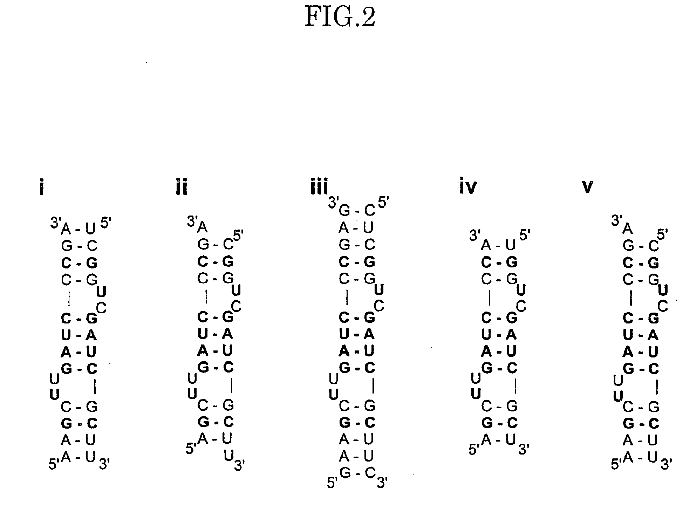

Specifically, in the kit of the present invention, the one oligonucleotide chain is the 5'-chain (left side chain in Fig. 2) or 3'-chain (right side chain in Fig. 2) of the modulate aptamer, and the other oligonucleotide is the 3'-chain or 5'-chain, respectively.

BRIEF DESCRIPTION OF THE DRAWINGS

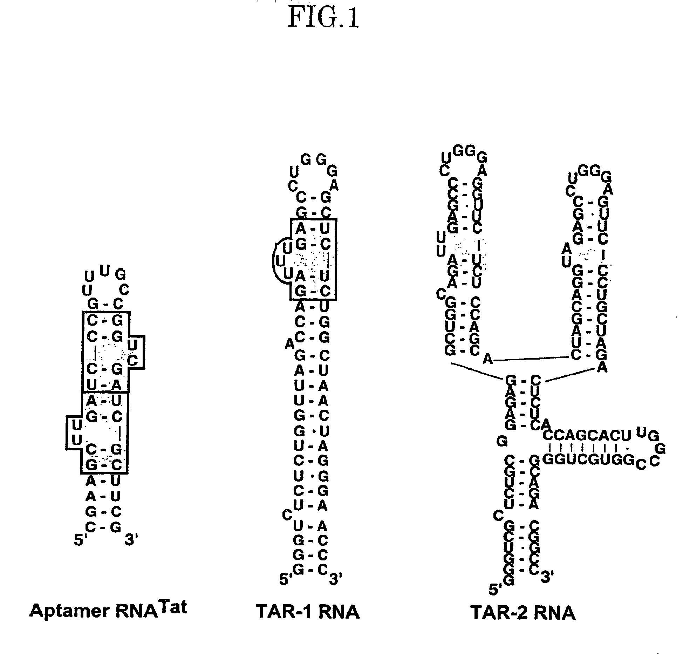

Figure 1 shows the secondary structures of aptamer RNA$^{Tat}$, TAR-1RNA (59mer) and TAR-2 RNA (123mer). The portion framed in solid lines shows the core factor necessary for Tat binding.

Figure 2 shows the secondary structures of the modulate aptamer R NAs of the present invention (i, DA-1/DA-2; ii, DA-3/DA-4; iii, DA-5/DA-6; iv, DA-7/DA-8; v, DA-1/DA-4).

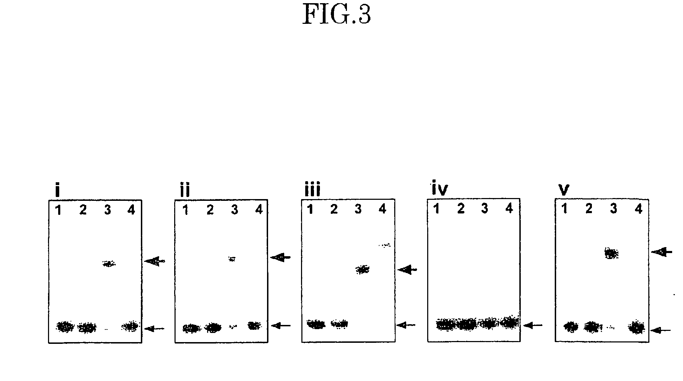

Figure 3 shows an autoradiogram of various modulate aptamer RNAs. Lane 1: radioactively labeled 5'-oligo (10 nM) only; lane 2: radioactively labeled 5'-oligo (10 nM) and unlabeled 3'-oligo (200 nM); lane 3: radioactively labeled 5'-oligo (10 nM) and unlabeled 3'-oligo (200 nM) in the presence of 20 nM CQ; lane 4: radioactively labeled 5'-oligo (10 nM) and unlabeled 3'-oligo (200 nM) in the presence of 200 nM Tat-1. A thick arrow shows the position of a modulate aptamer RNA-Tat or modulate aptamer RNA-CQ conjugate. The position of free 5'-oligonucleotide is shown with a thin arrow.

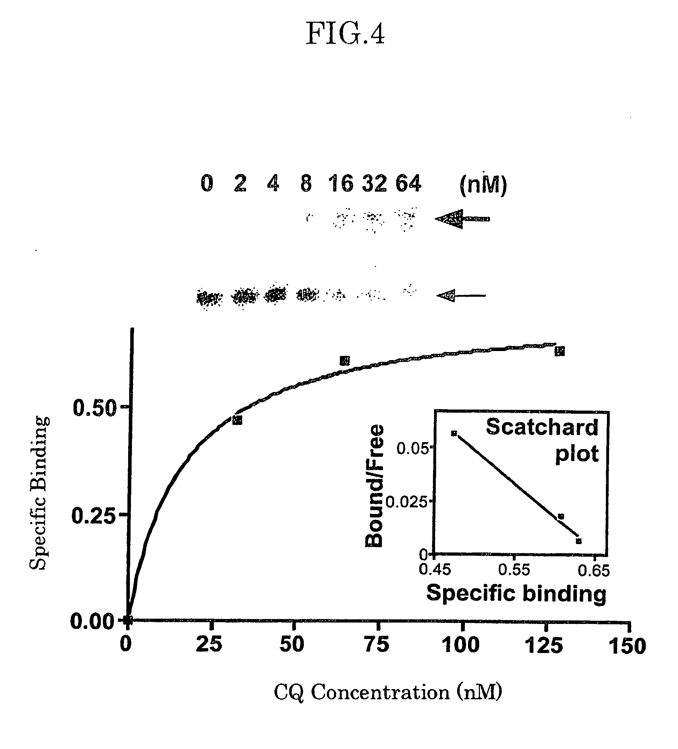

Figure 4 shows an autoradiogram obtained by a binding analysis using gel shift assay of modulate aptamer DA-1/DA-2 and CQ, and a saturation curve and Scatchard plot of modulate aptamer DA-1/DA-2-CQ. Thick arrow and thin a rrow show the positions of modulate aptamer RNA-CQ conjugate and free 5'-olig onucleotide, respectively.

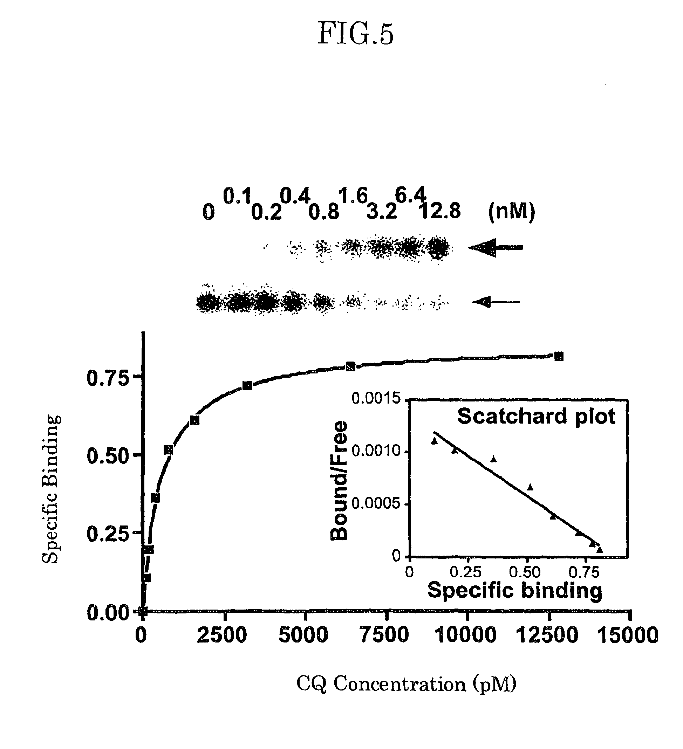

Figure 5 shows an autoradiogram obtained by a binding analysis using gel shift assay of modulate aptamer DA-5/DA-6 and CQ, and a saturation curve and Scatchard plot of modulate aptamer DA-5/DA-6-CQ. Thick arrow and thin a rrow show the positions of modulate aptamer RNA-CQ conjugate and free 5'-olig onucleotide, respectively.

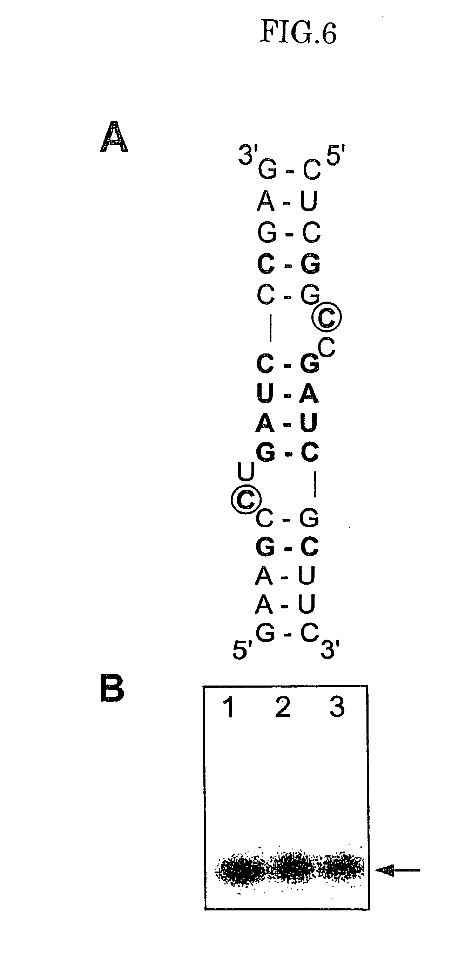

Figure 6 shows the secondary structure of an inactive-form modulate aptamer (DA-5i/DA-6i) (A) and an autoradiogram obtained by analysis of the binding of the aptamer with CQ peptide using gel shift assay (B). Lane 1: radioactively labeled 5'-oligo (10 nM); lane 2: radioactively labeled 5'-oligo (10 nM) and unlabeled 3'-oligo (200 nM); lane 3: radioactively labeled 5'-oligo (10 nM) and unlabeled 3'-oligo (200 nM) in the presence of 20 nM CQ.

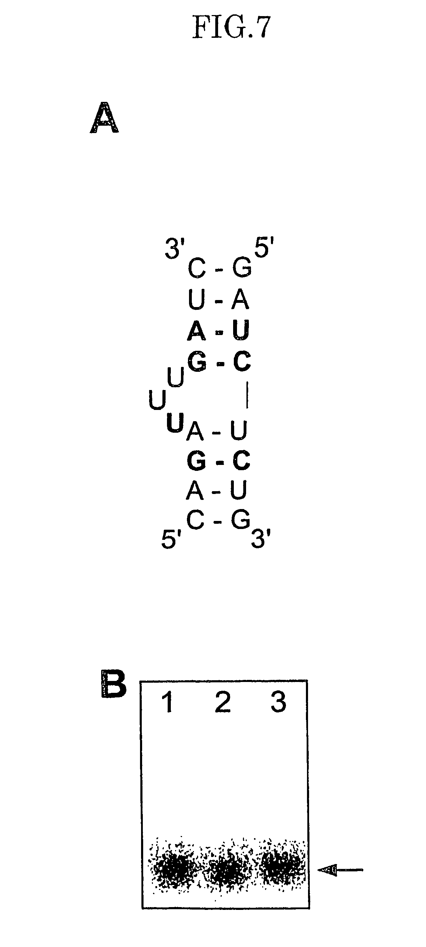

Figure 7 shows the secondary structure of double stranded TAR RNA (ii,DT-1/DT-2) (A) and an autoradiogram obtained by analysis of the binding of t he RNA with CQ peptide using gel shift assay (B). Lane 1: radioactively labeled 5'-oligo (10 nM); lane 2: radioactively labeled 5'-oligo (10 nM) and unlabeled 3'-oligo (200 nM); lane 3: radioactively labeled 5'-oligo (10 nM) and unlabeled 3'-oligo (200 nM) in the presence of 20 nM CQ.

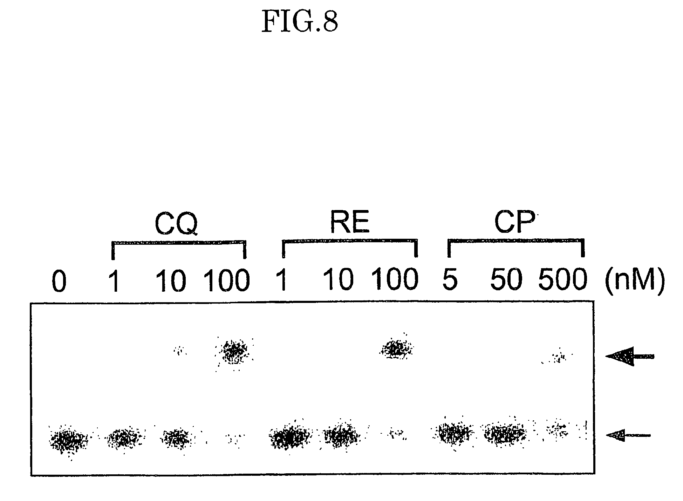

Figure 8 shows an autoradiogram obtained by a gel shift assay of the binding of modulate aptamer DA-5/DA-6 and Tat-derived peptides (CQ, RE, C P). Thick arrow and thin arrow show the positions of modulate aptamer RNA-CQ conjugate and free 5'-oligonucleotide, respectively.

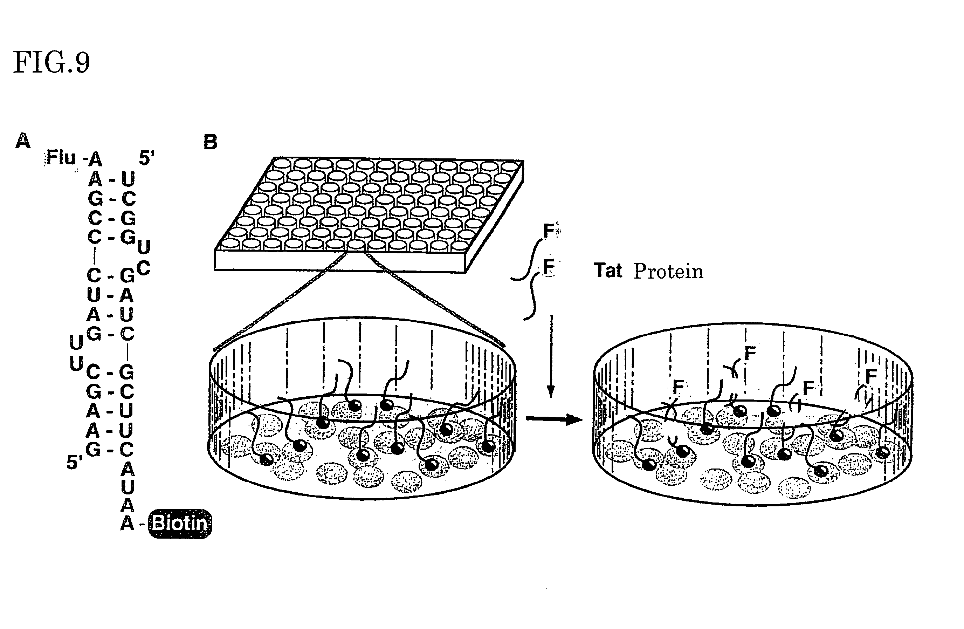

Figure 9 shows an analyte-dependent hybridizing oligonucleotide assay (ADHONA). This figure shows the secondary structure of modulate aptamer DA-9/DA-10 for use in ADHONA (A) and the mechanism of ADHONA (B)

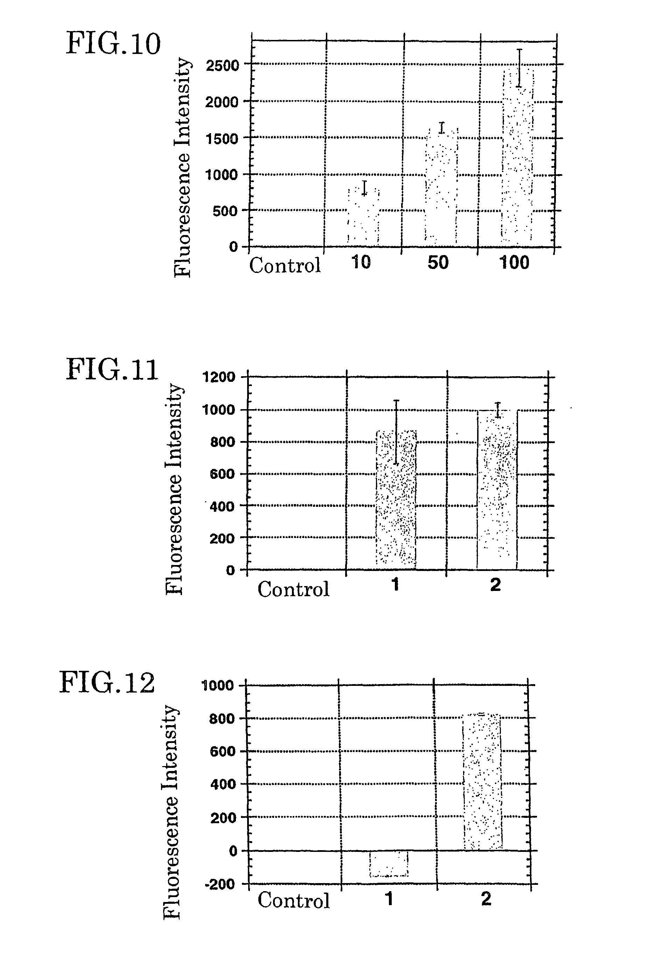

Figure 10 shows graphs indicating CQ peptide concentration dependency of ADHONA: fluorescent signal intensity in the absence of CQ peptide (control) or in the presence of CQ peptide (10, 50, 100 pmol). Results are indicated as an average ($\pm$ standard deviation) of 3 experiments.

Figure 11 shows the results of ADHONA using Tat-1 peptide: fluorescent signal intensity in the absence of Tat-1 or CQ peptide (control); fluorescent signal intensity in the presence of 10pmol CQ peptide (1) ; fluorescent signal intensity in the presence of 200pmol Tat-1 protein (2). Results are indicated as an average ($\pm$ standard deviation) of 3 experiments.

Figure 12 shows the influence on ADHONA by co-existence of HeLa nuclear extract: fluorescent signal intensity in the absence of Tat-1 or CQ peptide (control); fluorescent signal intensity in the presence of 8 units of HeLa nuclear extract (1); fluorescent signal intensity in the presence of 10pmol CQ peptide (2). Results are indicated as an average ($\pm$ standard deviation) of 3 experiments.

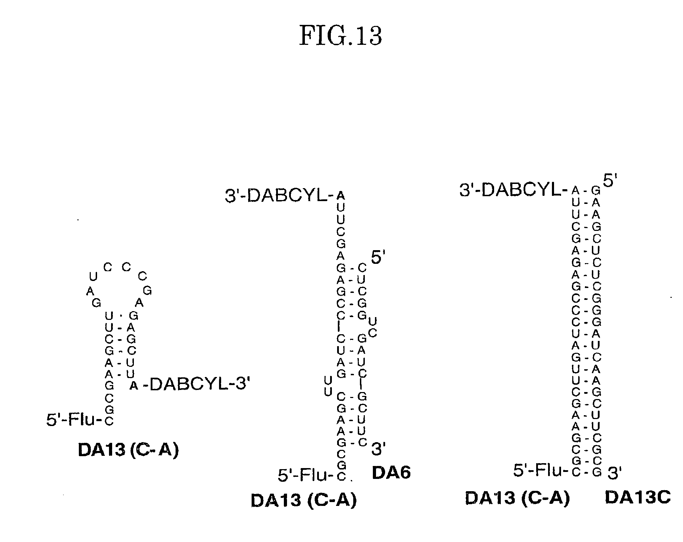

Figure 13 shows the secondary structure of oligonucleotide chain DA13(C-A) of a stem-loop structure having fluorescein bound to the 5'-end and DABCYL bound to the 3'-end, the secondary structure of the DA13(C-A)/DA6 which is a modulate aptamer RNA of the present invention, and nucleotide pair formation between DA13(C-A) and completely complementary DA13C.

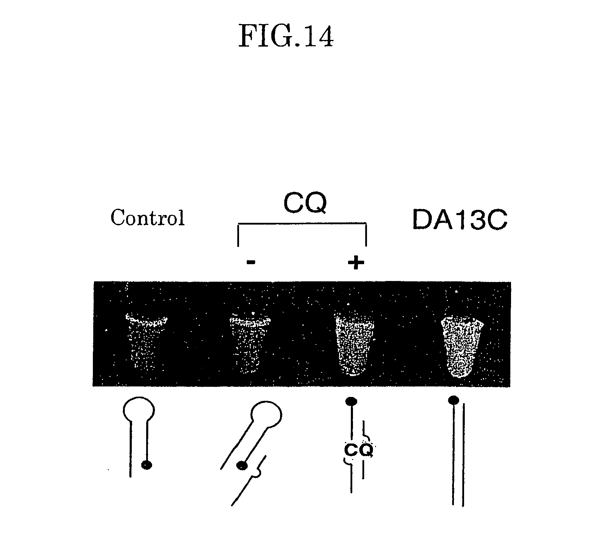

Figure 14 shows detection by fluorescence of CQ using a molecular beacon aptamer, and a model of the secondary structure of the oligonucleotide chain thereupon. 10nM DA13(C-A) only (Control); 10nM DA13(C-A)+100nM DA6 (CQ-); 10nM DA13(C-A)+100nM DA6+100nM CQ peptide (CQ+); 10nM DAl3(C-A)+100nM DAl3C(DA13C).

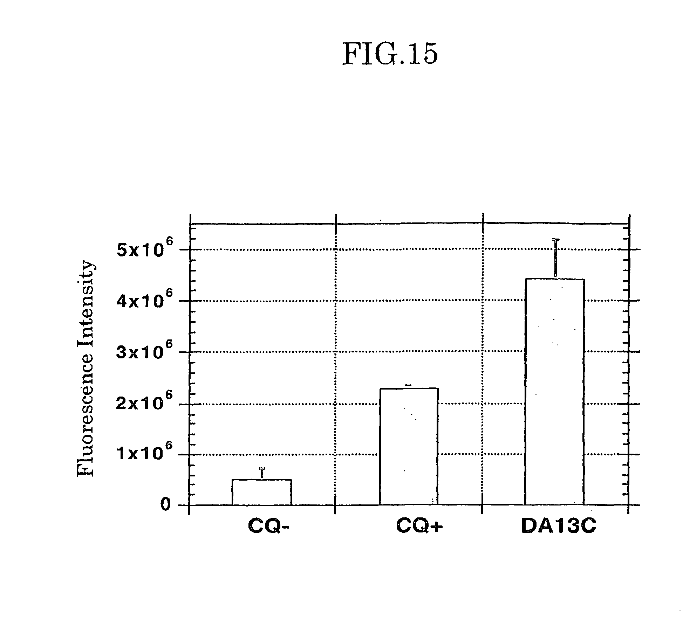

Figure 15, shows the result of measuring fluorescence intensity of samples CQ-, CQ+ and DA13C under the same conditions as for Fig. 14 using FluorImager (Molecular Dynamics). Results indicate the average ($\pm$standard deviation) of three experiments.

Explanation of sequence listing

SEQ ID NO:1: aptamer RNA for an HIV-1 Tat protein
SEQ ID NO:7: 5'- side oligonucleotide of a modulate aptamer
SEQ ID NO:8: 5'-side oligonucleotide of a modulate aptamer
SEQ ID NO:9: 5'- side oligonucleotide of a modulate aptamer
SEQ ID NO:10: 5'- side oligonucleotide of a modulate aptamer
SEQ ID NO:11: 3'-side oligonucleotide of a modulate aptamer
SEQ ID NO:12: 3'-side oligonucleotide of a modulate aptamer
SEQ ID NO:13: 3'-side oligonucleotide of a modulate aptamer
SEQ ID NO:14: 3'-side oligonucleotide of a modulate aptamer
SEQ ID NO:15: modified 5'-side nucleotide
SEQ ID NO:16: modified 3'-side nucleotide
SEQ ID NO:17: modified 5'-side nucleotide
SEQ ID NO:18: 5'- side oligonucleotide of a modulate aptamer
SEQ ID NO:19: 3'-side oligonucleotide of a modulate aptamer
SEQ ID NO:20: molecular beacon aptamer
SEQ ID NO:21: complementary oligonucleotide to a molecular beacon aptamer

BEST MODE FOR CARRYING OUT THE INVNETION

Below, the present invention will be explained in detail.

In the present invention, an "aptamer" refers to a nucleic acid ligand artificially engineered to bind strongly and specifically with a particular target protein. A "modulate aptamer" refers to an aptamer wherein the core portion of the aptamer is designed such that it has a low Tm value and divided in two short oligonucleotide chains which bind to form a double strand only in the presence of a target protein. A "molecular beacon aptamer" refers to an aptamer which comprises two structural components, a loop sequence which becomes a complementary probe to a target sequence, and a stem structure which is formed by annealing of two complementary arm sequences present on both ends of the probe sequence.

Further, in the present invention, "complementary" and "complementary base pair formation" refer to pairing between nucleobases: adenine and uracil, and guanine and cytosine by a hydrogen bond. Examples of nucleobases capable of complementary base pair formation include the combinations of adenine and uracil, and guanine and cytosine.

A "conjugate" refers to a formation in a stabilized state between a double stranded modulate aptamer according to the present invention and a target protein via hydrogen bonds and/or non-covalent bonds such as hydrophobic interactions. Here, "stabilized" refers to formation of a conjugate in which the binding-dissociation equilibrium is biased toward a bound state, and dissociation is difficult.

"Radioactive labeling" or "Radioactively label" refers to labeling using a substance including radioactive isotopes such as $^3$H, $^{13}$C, $^{32}$P, etc., and "non-radioactive labeling" or "non-radioactively label" refers to labeling not using radioactive substances. In non-radioactive labeling, labeling substances specifically include, but are not particular limited to luminescent molecules, fluorescent molecules such as fluorescein, enzymes such as peroxidase and alkali phosphatase, antibodies, and other molecules having binding specificity to specific molecules such as a biotin that are used in the art.

A "support" refers to objects used in the detection method and detection kit according to the present invention to perform conjugate formation reaction at an immobile position, and may be anything which does not exhibit absorbance such as glass or plastic. In the assay of the present invention using a minute amount of reaction solution, a microtiter plate and the like is particularly preferable.

A "target protein" refers to a protein or peptide which has high affinity and specificity for the modulate aptamer of the present invention, binds therewith and stabilizes, and the presence or absence, and/or amount thereof can be detected thereby. Examples include HIV-1 Tat and a fragment thereof having an amino acid chain length of approximately 40, CQ peptide, RE peptide, etc., and HIV Rev protein etc. Further, as HIV, HIV-1 and HIV-2 are known. Therefore, as used herein Tat-1 and TAR-1 respectively refer to HIV-1 Tat (trans activating factor) protein and TAR (trans activation response region) and Tat-2 and TAR-2 respectively refer to HIV-2 Tat protein and TAR.

The present inventors have previously isolated aptamer RNA$^{Tat}$ (Fig. 1 left, SEQ ID NO:1) which binds to HIV-1 Tat protein with higher specificity and affinity than HIV-1 TAR (Fig. 1 center), and reported the use thereof in a pharmaceutical composition for diagnosis, prevention and treatment of deseases in which HIV is involved (Yamamoto, R., Murakami, K., Taira, K. and Kumar, P.K.R., *Gene Ther. Mol. Biol.* **1**, 451-466 (1998), Patent Application Laid-Open No.: Hei 11-127864). This aptamer has high affinity for Tat protein (relative to Tat-1, Kd value is 100 times lower than TAR-1 RNA (59 mer, Fig. 1 center, SEQ ID NO:2), and relative to Tat-2, Kd value is 40 times lower than TAR-2 RNA (123 mer, Fig. 1 right, SEQ ID NO:3)), and since the sequence of the loop portion of the aptamer sequence is not the Tat protein binding site, it is possible to divide the aptamer into two strands.

From this viewpoint, the present inventors synthesized several modulate aptamer oligonucleotides having varying lengths and comprising core binding factor (the portion framed by solid lines in Fig. 1) relative to Tat-1 protein.

That is, first, the oligonucleotide chain corresponding to the 5'-side of the modulate aptamer of the present invention and the oligonucleotide chain corresponding to the 3'-side, were respectively chemically synthesized. Upon use, one or both of the two complementary oligonucleotide chains may be detectably labeled radioactively or non-radioactively as defined above and used.

The modulate aptamer of the present invention is reconstituted from the above oligonucleotide chains in the presence of a target protein. A preferable example thereof has the structure represented by the following secondary structure (I).

$$
\begin{array}{c}
3'-N^{6a}-N^{6b} \quad - \quad 5' \\
C \quad - \quad G \\
N^{5a}-N^{5b} \\
U \\
N^{4} \\
C \quad - \quad G \\
U \quad - \quad A \\
A \quad - \quad U \\
G \quad - \quad C \\
N^{3} \\
U \\
N^{2a}-N^{2b} \\
G \quad - \quad C \\
5'-N^{1a}-N^{1b} \quad - \quad 3'
\end{array}
\qquad (I)
$$

(In the structure, $N^{1a}$ and $N^{1b}$ represent at least 1 pair of nucleobases capable of complementary base pair formation; $N^{2a}$ and $N^{2b}$ represent at least 1 pair of nucleobases capable of complementary base pair formation; $N^3$ and $N^4$ each independently represent 1 or 2 nucleobases; $N^{5a}$ and $N^{5b}$ represent at least I pair of nucleobases capable of complementary base pair formation; $N^{6a}$ and $N^{6b}$ represent at least 1 pair of nucleobases capable of complementary base pair formation; and solid lines represent hydrogen bonds between nucleobases.)

The modulate aptamer of the present invention represented by the above secondary structure (I), exhibits high affinity to HIV-1 Tat protein by having the core factor of TAR-1 RNA repeated and arranged in the opposite direction, and binds sequence specifically to Tat-1 protein or Tat-derived peptides such as CQ, etc. Further, it was apparent that the presence of bulge residues protruding from the complementary base pair formation is essential for recognition of HIV-1 Tat protein and efficient binding.

Further, an example of one particularly preferable modulate aptamer in the present invention, is one having the nucleotide sequence represented by the following secondary structure (II):

3'        5'

G - C
A - U
G - C
C - G
C - G U
C - G C
U - A        ( II )
A - U
U G - C
U C - G
G - C
A - U
A - U
G - C

5'        3'

(In the structure, solid lines represent hydrogen bonds between nucleobases.)

From earlier biochemical experiments, the minimum necessary region in HIV-1 Tat protein for RNA recognition and binding was known. This peptide region called CQ peptide consists of amino acid residues 37-72 (SEQ ID NO:4), and binds to TAR-1 RNA with efficiency identical to that of full-length Tat-1 protein (Weeks, K.M., Ampe, C., Schultz, S.C., Steitz, T.A. and Crothers, D.M., *Science* **249,** 1281-1285 (1990); Calnan, B.J., Biancalnan, S., Hudson, D. and Frankel, A.D., *Genes Dev.* **5,** 201-210 (1991)). From this fact, experiments to examine the ability of a modulate aptamer were performed using Tat-1 protein or Tat-derived peptides such as CQ as a target protein. Further, as other peptides, RE derived from Tat-1 peptide (SEQ ID NO:5), CP derived from Tat-2 peptide (SEQ ID NO:6) were also used.

Further, in the present invention, there is provided a novel method of detecting a target protein using a modulate aptamer RNA which forms a conjugate only in the presence of the target protein. This method was analyzed particularly using HIV-1 Tat protein as a target. The modulate aptamer of the present invention form a conjugate efficiently in the presence of Tat protein, and does not form a conjugate in the presence of other RNA-binding proteins. Therefore, the presence and amount of Tat protein can be measured using the modulate aptamer.

In one embodiment of the present invention, one of the oligonucleotide chains of the modulate aptamer of the present invention is radioactively labeled as defined above, and a conjugate is formed in the presence of the other oligonucleotide chain and the target protein, and the presence or absence and/or amount of the conjugate formation is detected. Conditions for conjugate formation reaction are, for example, in a Tris buffer of pH 7 to 8, at 30°C, for 30 minutes, however, conditions such as reaction temperature, reaction time and the composition of the reaction solution can be modified as appropriate by a person skilled in the art.

After formation of a conjugate, it is possible to directly detect formation of the conjugate, however, in some cases, the formed conjugate may be separated by methods normally used in the art such as by electrophoresis.

A non-radioactive label may be used instead of the radioactive label, and for example where fluorescein is used, it can be detected by its fluorescent signal.

In another embodiment of the method of the present invention, one of the oligonucleotide chains is immobilized on a support as defined above, the conjugate formed by addition of the other radioactively or non-radioactively labeled chain in the presence of a target protein is detected. In this case, after conjugate formation reaction, oligonucleotides not forming conjugates can be removed by washing as required prior to detection. Immobilization may be performed by any method normally used in the art, such as by physical or chemical means, and there is no particular limitation on the method to be used. However, it is preferable to exploit specific binding between avidin or streptoavidin and biotin.

Further, the present invention provides a kit for detecting a target protein, which comprises the support, one oligonucleotide chain of the modulate aptamer to be immobilized on the support, and the other radioactively or non-radioactively labeled oligonucleotide chain which forms a conjugate in the presence of the target protein. Immobilization on the support may be performed at the time of use, however, a pre-immobilized oligonucleotide may be provided in a kit. Similarly to the above, immobilization may be of any type normally used in the art, and is not particularly limited, however, it is preferable to exploit specific binding between avidin or streptoavidin and biotin.

The specificity of the modulate aptamer of the present invention is preserved in a crude sample such as a HeLa nuclear extract, and the detection method of the present invention can be used directly in infected cells and the like.

By the present invention, it was possible to efficiently regulate the formation of a stable double stranded structure of aptamer-derived oligonucleotides in the presence of a target protein. Thereby, the present invention is the first to provide a method of detecting a target protein, particularly Tat protein, or a fragment thereof using a molecular beacon aptamer.

The molecular beacon aptamer used in the present invention, is used in the above described embodiment of a modulate aptamer, and is characterized in that one of the oligonucleotide chains constituting this modulate aptamer has mutually complementary nucleotide sequences consisting of four or more consecutive nucleotides, and has a stem-loop structure in the absence of a target protein. If the mutually complementary sequences consist of three nucleotides of less, a stable stem-loop structure cannot be adopted, which is not preferable.

One example of this stem-loop structure is one having the nucleotide sequence represented by the following secondary structure (III).

(In the structure, solid lines represent hydrogen bonds between nucleobases.)

In the present invention, when using a molecular beacon aptamer, a fluorescent substance is bound to the 5' or 3'-end of the oligonucleotide chain of the stem-loop structure, and a quencher substance against the fluorescent substance is bound to the 3' or 5' end, respectively.

Specifically, based on the sequence of the modulate aptamer, a quencher substance such as [4'-(4'-dimethyl-aminophenyl-azo) benzoic acid (DABSYL)] is connected by a covalent bond to 5' end of one of the oligonucleotide chains constituting the aptamer, i.e. the sequence which adopts a stem-loop structure, and to the 3'-end, a fluorescent substance such as fluorescein, cumarin, tetramethyl rhodamine etc., thereby constructing a molecular beacon aptamer (Fig. 13 left). As a control, an RNA oligonucleotide having a sequence that is completely complementary to this molecular beacon aptamer was synthesized, which can be used to evaluate the relative amount of fluorescence intensity when the fluorescent substance and the quencher substance are separated by a distance.

DA-13 (C-A) which constitutes one of the oligonucleotides of the modulate aptamer (Fig. 13 left), undergoes a structural change from a stem-loop structure to a double-stranded structure only in the presence of both the probe (the other chain of the modulate aptamer) and the target protein. In the absence of the probe or the target protein, this structural change does not occur. Therefore, in the present invention, the molecular beacon aptamer undergoes structural change in the presence of a target protein and this change can be detected efficiently.

The above described molecular beacon aptamer is one embodiment of the above described modulate aptamer, and it can be understood that the above described method and kit for detecting a target protein can be appropriately provided by using the molecular beacon aptamer in a similar manner.

Below, the present invention is further explained through the use of Examples, however, the present invention should not be taken to be limited by these Examples.

Example 1:

Synthesis of Tat-derived peptide, aptamer, and modulate aptamer

Chemical synthesis of each of Tat-1-derived peptide CQ (amino acids 37 to 72; SEQ ID NO:4), RE (amino acids 49 to 86; SEQ ID NO:5), and Tat-2-derived peptide CP (amino acids 66 to 97; SEQ ID NO:6) was entrusted to TANA Lab. L.C. (Texas, USA).

The aptamer RNA$^{Tat}$oligonucleotide (aptamer RNA, SEQ ID NO:1) shown in Fig. 1 was synthesized with an RNA/DNA synthesizer (Applied Biosystem model 394) using phosphoramidites (Glen Corporation, U.S.) and was deprotected and purified according to a known method described by the present inventors (Yamamoto, R., Murakami, K., Taira, K. and Kumar, P.K.R., *Gene Ther. Mol. Biol.* **1,** 451-466 (1998)).

On the other hand, oligonucleotide chains corresponding to the 5'-sid e of the modulate aptamer of the present invention (DA-1, DA-3, DA-5, and DA-7; SEQ ID NOS: 7, 8, 9, and 10, respectively), and oligonucleotide chains corres ponding to the 3'-side of said aptamer (DA-2, DA-4, DA-6, and DA-8, SEQ ID NOS: 11, 12, 13, and 14, respectively) were respectively synthesized and deprotec ted in the same manner as the above aptamer RNA. The 5'-chains of the modula te RNAs were $\gamma$-$^{32}$P-ATP-labeled with T4 polynucleotide kinase.

Example 2:

Gel shift assay

Using a gel shift assay, 5 types of modulate aptamers were examined for their conjugate formation.

Double strand formation of RNA oligonucleotides was analyzed in the presence of Tat or Tat-derived peptides, CQ, RE, or CP by a previously reported method (Yamamoto, R., Murakami, K., Taira, K. and Kumar, P.K.R., *Gene Ther. Mol. Biol.* **1**, 451-466(1998)).

Eight RNA oligonucleotides having the potential to form 5 types of double strand were analyzed (Fig. 2). In all cases, the 5'-chains of the double-stranded aptamer RNAs (DA-1,DA-3,DA-5,DA-7; SEQ ID NO:7, 8, 9, and 10, respectively) were $\gamma$-$^{32}$P-ATP-labeled. In 10$\mu$l of Tat-binding buffer (10 mM Tris-HCl, pH7.8, 70 mM NaCl, 2 mM EDTA, 0.01% Nonidet P-40), 5'-end labeled RNA (2 kcpm) and 200 nM of an unlabeled complementary chain RNA (relative to DA-1: DA-2 or DA-4; relative to DA-3: DA-4; relative to DA-5: DA-6; relative to DA-7: DA-8) were mixed in the presence of 40 nM *E. coli* tRNA.

To this was added 20 nM of CQ peptide or 200 nM of Tat protein, and the mixture was allowed to stand for 1 hour at 30°C. The reaction product was separated on non-denaturing polyacrylamide gel (15%), and the amount of conjugate formation in the presence or in the absence of the protein or peptide was measured with an image analyzer (BAS2000, Fujifilm, Japan).

Results are shown in Figure 3. Among the five types of oligonucleotide pairs, four pairs: DA-1/DA-2, DA-3/DA-4, DA-5/DA-6, and DA-1/DA-4 (i, ii, iii, and v, respectively, in Figs. 2 & 3), formed conjugates with high affinity in the presence of Tat protein (200 nM, lane 4) or Tat-derived peptide CQ (20 nM, lane 3) and did not form conjugates in the absence of Tat protein (lane 2). In particular, DA-5/DA-6 oligonucleotide pair formed a conjugate in the presence of Tat protein or CQ peptide more efficiently than the others, with conjugates with Tat at 50% and conjugates with CQ at 84% (Fig. 3iii).

Example 3:

Kinetic Analysis

The equilibrium dissociation constant (Kd) for conjugates of modulate aptamer RNA oligonucleotides DA-1/DA-2 (Fig. 2i) and DA-5/DA-6 (Fig. 2iii) obtained in Example 1, with CQ peptide was analyzed by gel shift assay in the presence of various concentrations of CQ (0.1 to 12.8 nM, 2 to 64 nM, respectively).

5'-end labeled RNA (50 pM) of DA-1 or DA-5, and RNA chains complementary thereto were mixed in a 10 $\mu$l Tat-binding buffer, and 40 nM tRNA was added as a non-specific competitor. CQ peptide (0.5 to 64 nM) was added and the mixture was allowed to stand for 1 hour at 30°C. The reaction product was separated on a non-denaturing polyacrylamide gel (15%), and analyzed. Values for $B_{max}$ and Kd were determined from the following binding equation:

$$Y = B_{max} \ x \ X/Kd+X$$

Y: Specific binding, $B_{max}$: maximum binding, X: ligand concentration

Non-linear regression analysis was performed with Graphpad PRISM software (Graphpad Software Inc, U.S.).

As is clear from the results shown in Figs. 4 and 5, Kd values for DA-5/DA-6-CQ, DA-1/DA-2-CQ conjugates were 0.5 nM and 400 nM, respectively, with the Kd value for the DA-1/DA-2-CQ conjugate being 800 times greater than that for the DA-5/DA-6-CQ conjugate. This difference is thought to be primarily caused by the two additional G-C base pair formations at both ends of the molecule in the DA-5/DA-6-CQ conjugate.

In other Tat peptides also, similar results were obtained. The modulate aptamers reconstituted a double-stranded RNA in the presence of Tat or CQ peptide, and exhibited affinity and specificity to the Tat protein comparable to that of hairpin aptamer RNA[Tat] (Fig. 1 left).

Example 4

Comparison with Core Factor Mutant-1

From the fact that the Kd value for the DA-5/DA-6-CQ conjugate approximated with the Kd value of a hairpin aptamer RNA[Tat], it is thought that modulate aptamers, particularly DA-5/DA-6, reconstitute a binding core factor in the presence of Tat protein or CQ peptide. The binding core factor of aptamer RNA[Tat] necessary for binding to the Tat protein consists of a central helix of four base pairs and two bulges comprising two residues each on each side thereof, and through site specific mutation experiments it was found that the U residues of both bulges were extremely important for binding to the Tat protein. Therefore, from kinetic analysis of the hairpin aptamer and modulate aptamer oligonucleotides DA-5/DA-6, these RNA compositions, i.e. both chains of DA-5 and DA-6 were predicted to interact with Tat protein.

In order to confirm this, the conjugate formation ability of a mutant (DA-5i/DA-6i (SEQ ID NO:15/16), DA-5i was labeled with $\gamma$-$^{32}$P-ATP) wherein the C residues of DA-5 and DA-6 were substituted by U residues (Figure 6A), was examined in the same manner as in Example 2. Results using CQ peptide are shown in Figure 6B. As is clear from the results of Figure 6B, this oligonucleotide pair (DA-5i/DA-6i) was unable to form a conjugate. This result indicated that functional groups important in the binding of the hairpin aptamer to Tat-1 (for example, the nitrogen at position 3 of a U residue), were also important in the binding of modulate aptamer DA-5/DA-6 to Tat-1.

Example 5

Comparison with Core Factor Mutant-2

In respect of a TAR-1 RNA-derived RNA oligonucleotide, DT-1/DT-2 (Fig. 7A, the sequence of DT-1 is represented by SEQ ID NO: 17) also, a comparison was made with DA-5/DA-6 in respect of binding to Tat-1 protein or CQ peptide in a manner similar to Example 2. Results using CQ peptide are shown in Fig. 7B. As is clear from the results in Fig. 7B, DT-1/DT-2 did not form a conjugate even in the presence of excess oligonucleotide (DT-2) and excess CQ.

From these results, it was clear that a modulate aptamer exhibits high affinity due to repetition and arrangement in the opposite direction of the TAR-1 RNA core factor, and binds sequence specifically with Tat protein and CQ peptide.

Example 6

Binding specificity to target protein

In the presence of HCV NS3 protein which is an RNA binding protein having a protease domain and an RNA helicase domain, a gel shift binding assay was performed with modulate aptamer oligonucleotides (DA-1/DA-2, DA-3/DA-4, DA-5/DA-6, DA-7/DA-8, DA-1/DA-4). In the gel shift analysis, this NS3 protein did not form a conjugate with the modulate aptamer oligonucleotide of the present invention which targets HIV-1 Tat protein (Data not shown).

Example 7

Affinity with HIV-2 Tat protein

In Tat proteins of HIV-1 and HIV-2, the core regions thereof (from cystein 36 to proline 57 of Tat-2) exhibit approximately 65% homology. In addition, from the research of the present inventors, it was found that an aptamer binds to Tat-2 peptide (CP, amino acids 66 to 97, SEQ ID NO:6) with greater affinity than TAR-2 RNA, and this fact indicated that the RNA binding characteristic of the two proteins was similar. Fig. 8 indicates that although efficiency is lower when compared with Tat-1 peptides CQ, RE, Tat-2 peptide CP also promotes formation of a double-strand of modulate aptamer DA-5/DA-6, respectively.

Example 8

Analyte (Tat)-dependent hybridizing oligonucleotide assay

The modulate aptamer of the present invention has potential as a tool for use in detection of a target protein such as Tat protein. A diagnostic assay such as indicated in Fig. 9 was developed and tested. In this analyte-dependent hybridizing oligonucleotide assay (analyte-dependent hybridizing oligonucleotide assay, ADHONA), a 3'-fluorescein oligonucleotide (DA-9, SEQ ID NO:18), a 3'-biotin oligonucleotide (DA-10, SEQ ID NO:19), and a streptoavidin-coated microtiter plate were used (Fig. 9A and B).

The streptoavidin-coated microtiter plate was purchased from Labsystems of Finland. 3'-biotinized oligonucleotide DA-10 (5 pmol) was placed in a streptoavidin plate (well), and left to stand at room temperature for 10 minutes in a 50μl of Tat binding buffer, and after allowing to bind with streptoavidin, the plate was washed with 200 μl of Tat binding buffer to wash away unbound oligonucleotides. Next, 50 μl of Tat binding buffer containing 10 pmol of 3'-fluorescein DA-9 and Tat-1 or Tat peptide (CQ or CP) was added, and the plate was allowed to stand for 30 minutes at 30°C, and finally washed again with 200 μ l Tat binding buffer to remove unbound substances. Fluorescence was analyzed with a fluoro image analyzer (FluorImager595). Excitation was at a wavelength of 488 nm, and detection at 530 nm. As a control, the same operations were performed in the absence of Tat-1 protein and Tat peptide.

Results are shown in Figs. 10 and 11. In the presence of 10 to 100 pmol of CQ peptide, fluorescence intensity increased to 500 to 2500 times that of the control (Fig. 10). Further, using Tat-1 protein (200 pmol), a fluorescence signal equivalent to that obtained with 10 pmol CQ peptide, was obtained. (Fig.11).

As is clear from the above results, conjugates were detected only in the presence of target protein, CQ peptide or Tat-1 protein, and the presence of the target protein was detected by using the modulate aptamer of the present invention.

Example 9

Reaction with co-existent Mammal cell nuclear extract

In order to be a diagnostic tool having good efficiency, in the assay sho wn in Fig. 9, it must be able to exhibit a quantitative result even in the presence of a crude sample such as a mammal cell nuclear extract. A crude sample may contain proteins and other mixtures which will interfere with the assay, and may further include proteins that promote annealing of a complementary sequence (Po rtman, D.S. and Dreyfuss, *G., EMBO J.* **13,** 213-221 (1994)). Here, the above ass ay was performed in the presence of DA-9/DA-10 with 8 units HeLa nuclear extr act (Promega, U.S.), and 40 units of RNase inhibitor (TOYOBO, Japan). Results are shown in Fig. 12. In the case where only HeLa nuclear extract was present, the modulate aptamer oligonucleotide did not form a double strand, and did not re main on the microtiter well (Even with 40 units of RNase inhibitor, a small amount of RNase activity remained).

This result indicated that the assay of the present invention was suitable for detecting Tat protein in the presence of mammalian cell nuclear extracts such as those in infected cells and the like.

Example 10

Use as a molecular beacon aptamer

As described above, formation of a stable double-stranded structure of aptamer-derived oligonucleotides such as DA-5 and DA-6 was able to be efficiently regulated by Tat or a Tat-derived peptide. Using this, the present invention was first able to provide a method for detecting Tat or a peptide thereof using a molecular beacon aptamer.

Based on a sequence of a modulate aptamer, a quencher substance [4'-(4'-dimethyl-aminophenyl-azo) benzoic acid (DABSYL)] was connected by a covalent bond to the 5'-end, and a fluorescent substance, fluorescein, was connected to the 3'-end of an oligonucleotide chain (SEQ ID NO:20) which has mutually complementary nucleotide sequences of 6 consecutive nucleotides within the molecule, which independently adopts a stem-loop structure and which can form a modulate aptamer by pairing with DA-6, thereby constructing a molecular beacon aptamer which was then synthesized (DA13(C-A), Fig. 13 left). Specifically, using phosphoramidite (Glen Corporation, U.S.), the oligonucleotide was synthesized with a RNA/DNA synthesizer (Applied Biosystem Model 394) and after 5'-fluoresceination and 3'-DABSYLation, deprotection was carried out according to methods established in the art. This complementary pair formation between DA13 (C-A) and DA-6 is indicated in Fig. 13. On the other hand, for the purpose of evaluating relative amounts of fluorescence intensity when the fluorescent substance and quencher substance are separated by a distance, DA-13C (SEQ ID NO:21) which has a sequence completely complementary to DA-13 (C-A) was synthesized using phosphoramidite (Glen Corporation, U.S.), with an RNA/DNA synthesizer (Applied Biosystem model 394) (Fig. 13 right).

Detection of Tat or a Tat peptide using a molecular beacon aptamer was performed as follows:

In a 0.5 ml tube, 10 nM of DA-13 (C-A) and 100 nM of DA-6 were mixed in 50μl of Tat binding buffer (10 mM Tris-HCl, pH 7.8, 70 mM NaCl, 2 mM EDTA, 0.01% Nonidet P-40) containing 40 nM of *E. Coli* tRNA, in the presence or absence of 100 nM of CQ peptide, and allowed to stand for 30 minutes at 30°C. The fluorescence intensity emitted as a result was measured with FluorImager (Molecular Dynamics). As a comparison, fluorescence intensity was measured under similar conditions in respect of 10 nM of DA-13 (C-A) only, or 10 nM DA-13 (C-A)/100 nM DA-13C.

As shown in the results in Figs. 14 and 15, DA-13 (C-A) itself adopted the stem-loop structure shown in Fig. 13 - left, and fluorescence was hardly detected. When both DA-6, and CQ being the target protein were present, this stem-loop structure underwent a structural change to a double-stranded structure, and fluorescence intensity increased dramatically. In the absence of DA-6 oligo or CQ, DA-13 (C-A) oligo did not undergo this structural change. On the other hand, DA-13 (C-A) oligonucleotide and DA-13C oligonucleotide formed a conjugate in Tat binding buffer even in the absence of CQ, and the fluorescence intensity thereof exhibit the maximum value. This was thought to be most likely due to the fluorescent substance binding portion and the quencher substance binding portion of DA-13(C-A)/DA-13C being separated by a distance 25 base pairs when compared with DA-13 (C-A)/DA-6 conjugate; and the DA-13 (C-A)/DA-13C conjugate being more stable than the DA-13 (C-A)/DA-6 conjugate.

As is clear from the above results, from the fact that fluorescent intensity increased markedly in the presence of DA-6 and CQ, it is apparent that molecular beacon [DA-13 (C-A)/DA-6] undergoes a structural change in the presence of the target protein: Tat or Tat peptide, and Tat can be efficiently detected by monitoring this structural change through fluorescence measurement.

INDUSTRIAL APPLICABILITY

The modulate aptamer of the present invention has the following numerous advantages when compared to known conventional long non-modulate aptamers.

1) Short RNA oligonucleotides can be synthesized with higher efficiency than long ones.
2) Modification for stabilizing nucleic acids may be partial in the aptamer.
3) Low cost.
4) Suitable structuring of the modulate aptamer is promoted by the analyte.

Further, the above results indicate that the method of detecting a protein with the modulate aptamer of the present invention has high sensitivity and high specificity, and can be easily applied to nucleic acid array analysis techniques having complex steps. Further, when the RNA aptamer of the present invention is completely protected against ribonuclease, the method will be even more effective. The method of the present invention can detect not only HIV Tat protein, but can be generally applied to the detection of other proteins such as HIV Rev protein using a modulate aptamer for RRE (Rev response element) by selecting suitable non-modulate and modulate-type aptamers from a combined library.

Further, in respect also of the molecular beacon aptamer which is one embodiment of the modulate aptamer of the present invention, it is possible to make modification such that the aptamers have resistance against nuclease, thereby allowing these stabilized molecular beacon aptamers to be applied for the detection of Tat protein in living cells (for example, in HIV infected cells).

## Claims

1. A modulate aptamer being an aptamer constituted by two divided complementary oligonucleotide chains, which forms a conjugate and stabilises only in the presence of a target protein, wherein one or both of the two oligonucleotide chains is radioactively or non-radioactively labeled.

2. The modulate aptamer according to claim 1, wherein one of the oligonucleotide chains constituting the modulate aptamer has intramolecularly, mutually complementary sequences of four or more consecutive nucleotides and has a stem-loop structure in the absence of a target protein.

3. The modulate aptamer according to claim 2, wherein a fluorescent substance is bound to the 5' or 3'-end of the oligonucleotide of the stem-loop structure, and a quencher substance for the fluorescent substance is bound to the 3' or 5' end thereof, respectively.

4. The modulate aptamer according to any one of claims 1 to 3, wherein the target protein is HIV-1 Tat protein and/or a fragment thereof.

5. The modulate aptamer according to claim 4, which comprises the nucleotide sequence represented by the following secondary structure (I):

$$
\begin{array}{l}
3' - N^{6a} - N^{6b} \quad - \quad 5' \\
\quad\quad C \; - \; G \\
\quad\quad N^{5a} - N^{5b} \\
\quad\quad\quad\quad\quad U \\
\quad\quad\quad\quad\quad\; N^{4} \\
\quad\quad C \; - \; G \\
\quad\quad U \; - \; A \\
\quad\quad A \; - \; U \\
\quad\quad G \; - \; C \\
\quad N^{3} \\
\quad U \\
\quad\quad N^{2a} - N^{2b} \\
\quad\quad G \; - \; C \\
5' - N^{1a} - N^{1b} \quad - \quad 3'
\end{array}
\qquad (I)
$$

(In the structure, $N^{1a}$ and $N^{1b}$ represent at least 1 pair of nucleobases capable of complementary base pair formation; $N^{2a}$ and $N^{2b}$ represent at least 1 pair of nucleobases capable of complementary base pair formation; $N^{3}$ and $N^{4}$ each independently represent 1 or 2 nucleobases; $N^{5a}$ and $N^{5b}$ represent at least 1 pair of nucleobases capable of complementary base pair formation; $N^{6a}$ and $N^{6b}$ represent at least 1 pair of nucleobases capable of complementary base pair formation; and solid lines represent hydrogen bonds between nucleobases.)

6. The modulate aptamer according to claim 5, which comprises the nucleotide sequence represented by the following

secondary structure:

```
          3'      5'

          G - C
          A - U
          G - C
          C - G
          C - G U
          C - G C
          U - A            (II)
          A - U
        U G - C
        U C - G
          G - C
          A - U
          A - U
          G - C


          5'      3'
```

(In the structure, solid lines represent hydrogen bonds between nucleobases.)

7. The modulate aptamer according to claim 2, wherein one of the oligonucleotide chains constituting the modulate aptamer has a nucleotide sequence represented by the following secondary structure (III):

```
              C   C
          U           C
        A               G
          G           A
            U - G
            U - A
            C - G            (III)
            G - C
            A - U
            A - U
            G     A — 3'
            C
            G
      5' — C
```

(In the structure, solid lines represent hydrogen bonds between nucleobases.)

8. A method of detecting a target protein, which comprises radioactively or non-radioactively labeling one oligonucleotide chain of the modulate aptamer according to claim 1, and detecting the presence and/or amount of the target protein with a conjugate formed in the presence of the target protein as an indicator.

9. A method of detecting a target protein, which comprises immobilizing one oligonucleotide chain of the modulate aptamer according to claim 1 on a support, and detecting the presence and/or amount of a target protein with a conjugate formed by addition of the other oligonucleotide labeled radioactively or non-radioactively as an indicator.

10. The method according to claim 8 or 9, wherein the oligonucleotide chain of the stem-loop structure according to claim 3 is used as the oligonucleotide chain labeled.

11. The method according to claim 9 or 10, wherein the immobilization is by specific binding between avidin or streptoavidin and biotin.

12. The method according to claim 8 or 9, wherein the non-radioactive label is fluorescein and the conjugate is detected by the fluorescent signal thereof.

13. The method according to any one of claims 8 to 12, wherein the target protein is an HIV-1 Tat protein and/or a fragment thereof.

14. A kit for detecting a target protein, which comprises the following (a) to (c):

    (a) a support,
    (b) one of the oligonucleotide chains of the modulate aptamer according to claim 1 to be immobilized on the support,
    (c) the other oligonucleotide chain which is radioactively or non-radioactively labeled and forms a conjugate in the presence of a target protein.

15. The kit for detecting a target protein according to claim 14, wherein the target protein is an HIV-1 Tat protein and/or a fragment thereof.

16. The kit for detecting a target protein according to claim 14, wherein one of the oligonucleotide chain (b) is the 5'-chain or the 3'-chain of the modulate aptamer according to claim 5 or 6, and the other oligonucleotide chain (c) is the 3'-chain or the 5'chain, respectively.

17. The kit for detecting a target protein according to claim 14 or 15, wherein the oligonucleotide chain of the stem-loop structure according to claim 3 is used as the labeled oligonucleotide chain.

**Patentansprüche**

1. Modul-Adaptamer, welches ein Adaptamer ist, das aus zwei geteilten komplementären Oligonukleotidketten besteht und nur in Gegenwart eines Zielproteins ein Konjugat bildet und sich stabilisiert, wobei eine oder beide der zwei Oligonukleotidketten radioaktiv oder nicht-radioaktiv markiert ist bzw. sind.

2. Modul-Adaptamer nach Anspruch 1, wobei eine der Oligonukleotidketten, aus denen das Modul-Adaptamer besteht, intramolekulare, wechselseitig komplementäre Sequenzen von vier oder mehreren aufeinanderfolgenden Nukleotiden aufweist und in Abwesenheit eines Zielproteins eine Stem-Loop-Struktur hat.

3. Modul-Adaptamer nach Anspruch 2, wobei eine fluoreszierende Substanz an das 5'- oder 3'-Ende des Oligonukleotids mit der Stem-Loop-Struktur gebunden ist und eine Quencher-Substanz für die fluoreszierende Substanz an das 3'- bzw. das 5'-Ende davon gebunden ist.

4. Modul-Adaptamer nach einem der Ansprüche 1 bis 3, wobei das Zielprotein HIV-1-Tat-Protein und/oder ein Fragment davon ist.

**5.** Modul-Adaptamer nach Anspruch 4, welches die durch die folgende Sekundärstruktur (I) repräsentierte Nukleotidsequenz umfaßt:

$$
\begin{array}{l}
3' - N^{6a} - N^{6b} \quad - \quad 5' \\
\qquad\quad C \;-\; G \\
\qquad N^{5a} - N^{5b} \\
\qquad\qquad\qquad U \\
\qquad\qquad\qquad\quad N^{4} \\
\qquad\quad C \;-\; G \\
\qquad\quad U \;-\; A \\
\qquad\quad A \;-\; U \\
\qquad\quad G \;-\; C \\
\qquad N^{3} \\
\qquad U \\
\qquad\quad N^{2a} - N^{2b} \\
\qquad\quad G \;-\; C \\
5' - N^{1a} - N^{1b} \quad - \quad 3'
\end{array}
\qquad (I)
$$

(In der Struktur repräsentieren $N^{1a}$ und $N^{1b}$ wenigstens 1 Paar von Nukleobasen, die zur Bildung komplementärer Basenpaare in der Lage sind, $N^{2a}$ und $N^{2b}$ repräsentieren wenigstens 1 Paar von Nukleobasen, die zur Bildung komplementärer Basenpaare in der Lage sind, $N^{3}$ und $N^{4}$ repräsentieren jeweils unabhängig voneinander 1 oder 2 Nukleobasen, $N^{5a}$ und $N^{5b}$ repräsentieren wenigstens 1 Paar von Nukleobasen, die zur Bildung komplementärer Basenpaare in der Lage sind, $N^{6a}$ und $N^{6b}$ repräsentieren wenigstens 1 Paar von Nukleobasen, die zur Bildung komplementärer Basenpaare in der Lage sind, und durchgezogene Linien repräsentieren Wasserstoffbindungen zwischen Nukleobasen.)

**6.** Modul-Adaptamer nach Anspruch 5, welches die durch die folgende Sekundärstruktur repräsentierte Nukleotidsequenz umfaßt:

$$3' \qquad 5'$$

```
        G - C
        A - U
        G - C
        C - G
        C - G  U
        C - G C
        U · A
        A - U
     U  G · C
     U  C - G              (Ⅱ)
        G - C
        A - U
        A - U
        G - C
```

$$5 \qquad 3'$$

(In der Struktur repräsentieren durchgezogene Linien Wasserstoffbindungen zwischen Nukleobasen.)

7. Modul-Adaptamer nach Anspruch 2, wobei eine der Oligonukleotidketten, aus denen das Modul-Adaptamer besteht, eine Nukleotidsequenz hat, die durch die nachfolgende Sekundärstruktur (III) repräsentiert wird:

```
            C   C
        U           C
     A                 G
       G             A
         U ‑ G
         U ‑ A
         C ‑ G          (Ⅲ)
         G ‑ C
         A ‑ U
         A ‑ U
         G     A ‑3'
         C
         G
   5'‑ C
```

(In der Struktur repräsentieren durchgezogene Linien Wasserstoffbindungen zwischen Nukleobasen.)

8.  Verfahren zum Detektieren eines Zielproteins, umfassend das radioaktive oder nicht-radioaktive Markieren einer Oligonukleotidkette des Modul-Adaptamers nach Anspruch 1 und das Detektieren des Vorhandenseins und/oder der Menge des Zielproteins mit einem in Gegenwart des Zielproteins als Indikator gebildeten Konjugat.

9.  Verfahren zum Detektieren eines Zielproteins, umfassend das Immobilisieren einer Oligonukleotidkette des Modul-Adaptamers nach Anspruch 1 auf einem Träger und das Detektieren des Vorhandenseins und/oder der Menge eines Zielproteins mit einem durch die Zugabe des anderen radioaktiv oder nicht-radioaktiv markierten Oligonukleotids als Indikator gebildeten Konjugat.

10. Verfahren nach Anspruch 8 oder 9, wobei die Oligonukleotidkette mit der Stem-Loop-Struktur nach Anspruch 3 als die markierte Oligonukleotidkette verwendet wird.

11. Verfahren nach Anspruch 9 oder 10, wobei die Immobilisierung durch spezifische Bindung zwischen Avidin oder Streptavidin und Biotin erfolgt.

12. Verfahren nach Anspruch 8 oder 9, wobei die nicht-radioaktive Markierung Fluorescein ist und das Konjugat durch dessen Fluoreszenzsignal detektiert wird.

13. Verfahren nach einem der Ansprüche 8 bis 12, wobei das Zielprotein ein HIV-1-Tat-Protein und/oder ein Fragment davon ist.

14. Kit zum Detektieren eines Zielproteins, welcher die folgenden (a) bis (c) umfaßt:

   (a) einen Träger,
   (b) eine der Oligonukleotidketten des Modul-Adaptamers nach Anspruch 1 zur Immobilisierung auf dem Träger,
   (c) die andere Oligonukleotidkette, die radioaktiv oder nicht-radioaktiv markiert ist und in Gegenwart eines Zielproteins ein Konjugat bildet.

**15.** Kit zum Detektieren eines Zielproteins nach Anspruch 14, wobei das Zielprotein ein HIV-1-Tat-Protein und/oder ein Fragment davon ist.

**16.** Kit zum Detektieren eines Zielproteins nach Anspruch 14, wobei eine der Oligonukleotidketten (b) die 5'-Kette oder die 3'-Kette des Modul-Adaptamers nach Anspruch 5 oder 6 ist und die andere Oligonukleotidkette (c) die 3'-Kette bzw. die 5'-Kette ist.

**17.** Kit zum Detektieren eines Zielproteins nach Anspruch 14 oder 15, wobei die Oligonukleotidkette mit der Stem-Loop-Struktur nach Anspruch 3 als die markierte Oligonukleotidkette verwendet wird.

**Revendications**

**1.** Aptamère module, qui est un aptamère constitué par deux chaînes d'oligonucléotides complémentaires dissociées, qui forme un conjugué et se stabilise uniquement en présence d'une protéine cible, dans lequel l'une des deux chaînes d'oligonucléotides ou les deux chaînes est (sont) marquée(s) radioactivement ou non radioactivement.

**2.** Aptamère module selon la revendication 1, dans lequel l'une des chaînes d'oligonucléotides constituant l'aptamère module possède intramoléculairement des séquences mutuellement complémentaires de quatre nucléotides consécutifs ou davantage et possède une structure en épingle à cheveux en l'absence d'une protéine cible.

**3.** Aptamère module selon la revendication 2, dans lequel une substance fluorescente est liée à l'extrémité 5' ou à l'extrémité 3' de l'oligonucléotide de la structure en épingle à cheveux, et une substance d'extinction de la substance fluorescente est liée à son extrémité 3' ou 5', respectivement.

**4.** Aptamère module selon l'une quelconque des revendications 1 à 3, dans lequel la protéine cible est une protéine Tat du VIH-1 et/ou un fragment de celle-ci.

**5.** Aptamère module selon la revendication 4, qui comprend la séquence de nucléotides représentée par la structure secondaire (I) suivante :

$$
\begin{array}{l}
3' - N^{6a} - N^{6b} \quad - \quad 5' \\
\quad\; C \; - \; G \\
\quad\; N^{5a} - N^{5b} \\
\qquad\qquad\quad U \\
\qquad\qquad\quad N^{4} \\
\quad\; C \; - \; G \\
\quad\; U \; - \; A \\
\quad\; A \; - \; U \\
\quad\; G \; - \; C \\
\; N^{3} \\
\; U \\
\quad\; N^{2a} - N^{2b} \\
\quad\; G \; - \; C \\
5' - N^{1a} - N^{1b} \quad - \quad 3'
\end{array} \qquad (I)
$$

(Dans la structure, $N^{1a}$ et $N^{1b}$ représentent au moins une paire de nucléobases capables de formation d'une paire de bases complémentaires ; $N^{2a}$ et $N^{2b}$ représentent au moins une paire de nucléobases capables de formation d'une paire de bases complémentaires ; $N^3$ et $N^4$ représentent chacun indépendamment 1 ou 2 nucléobases ; $N^{5a}$ et $N^{5b}$ représentent au moins une paire de nucléobases capables de formation d'une paire de bases complémentaires ; $N^{6a}$ et $N^{6b}$ représentent au moins une paire de nucléobases capables de formation d'une paire de bases complémentaires ; et les traits continus représentent des liaisons hydrogène entre les nucléobases).

6. Aptamère module selon la revendication 5, qui comprend la séquence de nucléotides représentée par la structure secondaire suivante :

<div align="center">

3'      5'

G - C
A - U
G - C
C - G
C - G U
C - G C
U - A     ( II )
A - U
U G - C
U C - G
G - C
A - U
A - U
G - C

5'      3'

</div>

(Dans la structure, les traits continus représentent des liaisons hydrogène entre les nucléobases).

7. Aptamère module selon la revendication 2, dans lequel l'une des chaînes d'oligonucléotides constituant l'aptamère module possède une séquence de nucléotides représentée par la structure secondaire (III) suivante :

```
              C   C
          U        C
        A            G
          G        A
            U · G
            U - A
            C - G          (Ⅲ)
            G - C
            A - U
            A - U
            G   A —3'
            C
            G
       5'—  C
```

(Dans la structure, les traits continus représentent des liaisons hydrogène entre les nucléobases).

**8.** Procédé de détection d'une protéine cible, qui comprend le marquage radioactif ou non radioactif d'une chaîne d'oligonucléotides de l'aptamère module selon la revendication 1, et la détection de la présence et/ou de la quantité de la protéine cible avec un conjugué formé en présence de la protéine cible en tant qu'indicateur.

**9.** Procédé de détection d'une protéine cible, qui comprend l'immobilisation d'une chaîne d'oligonucléotides de l'aptamère module selon la revendication 1 sur un support, et la détection de la présence et/ou de la quantité d'une protéine cible avec un conjugué formé par addition de l'autre oligonucléotide marqué radioactivement ou non radioactivement en tant qu'indicateur.

**10.** Procédé selon la revendication 8 ou 9, dans lequel la chaîne d'oligonucléotides de la structure en épingle à cheveux selon la revendication 3 est utilisée en tant que chaîne d'oligonucléotides marquée.

**11.** Procédé selon la revendication 9 ou 10, dans lequel l'immobilisation intervient par liaison spécifique entre une avidine ou une streptavidine et une biotine.

**12.** Procédé selon la revendication 8 ou 9, dans lequel le marqueur non radioactif est la fluorescéine et le conjugué est détecté par le signal fluorescent de celle-ci.

**13.** Procédé selon l'une quelconque des revendications 8 à 12, dans lequel la protéine cible est une protéine Tat du VIH-1 et/ou un fragment de celle-ci.

**14.** Trousse pour la détection d'une protéine cible, qui comprend ce qui suit de (a) à (c) :

(a) un support,
(b) l'une des chaînes d'oligonucléotides de l'aptamère module selon la revendication 1 à immobiliser sur le support,
(c) l'autre chaîne d'oligonucléotides qui est marquée radioactivement ou non radioactivement et forme un conjugué en présence d'une protéine cible.

**15.** Trousse pour la détection d'une protéine cible selon la revendication 14, dans laquelle la protéine cible est une protéine Tat du VIH-1 et/ou un fragment de celle-ci.

**16.** Trousse pour la détection d'une protéine cible selon la revendication 14, dans laquelle l'une des chaînes d'oligonucléotides (b) est la chaîne 5' ou la chaîne 3' de l'aptamère module selon la revendication 5 ou 6, et l'autre chaîne d'oligonucléotides (c) est, respectivement, la chaîne 3' ou la chaîne 5'.

**17.** Trousse pour la détection d'une protéine cible selon la revendication 14 ou 15, dans laquelle la chaîne d'oligonucléotides de la structure en épingle à cheveux selon la revendication 3 est utilisée en tant que chaîne d'oligonucléotides marquée.

# FIG.1

**Aptamer RNA**Tat    **TAR-1 RNA**    **TAR-2 RNA**

# FIG.2

# FIG.3

# FIG.4

# FIG.5

# FIG.6

## FIG.7

**A**

```
   3'        5'
     C - G
     U - A
     A - U
    U G - C
  U U      |
   U A - U
     G - C
     A - U
     C - G
   5'        3'
```

**B**

# FIG.8

FIG.9

FIG.10

FIG.11

FIG.12

# FIG.13

# FIG.14

# FIG.15

SEQUENCE LISTING

<110> Japan as represented by Secretary of Agency of Industrial Science and Technology

<120> Modulate aptamers and the method of detecting target proteins using them

<130> PH-933-PCT

<160> 21

<170> PatentIn Ver. 2.0

<210> 1
<211> 35
<212> RNA
<213> Artificial Sequence

<220>
<223> aptamer RNA for HIV-1 Tat protein

<400> 1
cgaagcuuga ucccguuugc cggucgaucg cuucg          35

<210> 2
<211> 59
<212> RNA
<213> Homo sapiens

<400> 2
gggucucucu gguuagacca gauuugagcc ugggagcucu cuggcuaacu agggaaccc          59

<210> 3
<211> 124
<212> RNA
<213> Homo sapiens

<400> 3

gggucgcucu gcggagaggc uggcagauug agcccuggga gguucucucc agcacuagca 60

gguagagccu gggaguuccc ugcuagacuc ucaccagcac uuggccggug cugggcagac 120

ggcc                                                                       124

<210> 4
<211> 36
<212> PRT
<213> Homo sapiens

<400> 4

Cys Phe Thr Thr Lys Ala Leu Gly Ile Ser Tyr Gly Arg Lys Lys Arg
1           5            10            15

Arg Gln Arg Arg Arg Pro Pro Gln Gly Ser Gln Thr His Gln Val Ser
     20           25           30

Leu Ser Lys Gln
      35

<210> 5
<211> 38
<212> PRT
<213> Homo sapiens

<400> 5

Arg Lys Lys Arg Arg Gln Arg Arg Arg Pro Pro Gln Gly Ser Gln Thr
1          5            10            15

His Gln Val Ser Leu Ser Lys Gln Pro Thr Ser Gln Ser Arg Gly Asp
     20           25           30

Pro Thr Gly Pro Lys Glu
      35

<210> 6
<211> 32
<212> PRT
<213> Homo sapiens

<400> 6

Cys Phe Leu Asn Lys Gly Leu Gly Ile Cys Tyr Glu Arg Lys Gly Arg

1                   5                         10                        15

Arg Arg Arg Thr Pro Lys Lys Thr Lys Thr His Pro Ser Pro Thr Pro

                  20                          25                         30

<210> 7
<211> 14
<212> RNA
<213> Artificial Sequence

<220>
<223> 5'-half oligonucleotide of modulate aptamer

<400> 7
aagcuugauc ccga          14

<210> 8
<211> 13
<212> RNA
<213> Artificial Sequence

<220>
<223> 5'-half oligonucleotide of modulate aptamer

<400> 8
agcuugaucc cga           13

<210> 9
<211> 16
<212> RNA
<213> Artificial Sequence

<220>
<223> 5'-half oligonucleotide of modulate aptamer

<400> 9
gaagcuugan cccgag         16

<210> 10
<211> 12
<212> RNA
<213> Artificial Sequence

<220>
<223> 5'-half oligonucleotide of modulate aptamer

<400> 10
agcuugaucc ca            12

<210> 11
<211> 14

<212> RNA
<213> Artificial Sequence

<220>
<223> 3'-half oligonucleotide of modulate aptamer

<400> 11
ucggucgauc gcuu          14

<210> 12
<211> 13
<212> RNA
<213> Artificial Sequence

<220>
<223> 3'-half oligonucleotide of modulate aptamer

<400> 12
cggucgaucg cuu          13

<210> 13
<211> 16
<212> RNA
<213> Artificial Sequence

<220>
<223> 3'-half oligonucleotide of modulate aptamer

<400> 13
cucggucgau cgcuuc          16

<210> 14
<211> 12
<212> RNA
<213> Artificial Sequence

<220>
<223> 3'-half oligonucleotide of modulate aptamer

<400> 14
uggucgaucg cu          12

<210> 15
<211> 16
<212> RNA
<213> Artificial Sequence

<220>
<223> altered 5'-half oligonucleotide

<400> 15
gaagccugau cccgag          16

<210> 16
<211> 16
<212> RNA
<213> Artificial Sequence

<220>
<223> altered 3'-half oligonucleotide

<400> 16
cucggccgau cgcuuc          16

<210> 17
<211> 11
<212> RNA
<213> Artificial Sequence

<220>
<223> altered 5'-half oligonucleotide

<400> 17
cagauuugau c          11

<210> 18
<211> 16
<212> RNA
<213> Artificial Sequence

<220>
<223> 5'-half oligonucleotide of modulate aptamer

<400> 18
gaagcuugau cccgaa          16

<210> 19
<211> 19
<212> RNA
<213> Artificial Sequence

<220>
<223> 3'-half oligonucleotide of modulate aptamer

<400> 19
ucggucgauc gcuucauaa          19

<210> 20
<211> 25
<212> RNA
<213> Artificial Sequence

<220>
<223> molecular beacon aptamer

<400> 20
cgcgaagcuu gaucccgaga gcuua          25

<210> 21
<211> 25
<212> RNA
<213> Artificial Sequence

<220>
<223> oligonucleotide complementary to molecular beacon aptamer

<400> 21
gaagcucucg ggaucaagcu ucgcg     25